# EUROPEAN PATENT SPECIFICATION

(11) **EP 0 930 297 B1**
(45) Date of publication and mention of the grant of the patent: **23.04.2003**
(21) Application number: 99300183.3
(22) Date of filing: 12.01.1999
(51) Int. Cl.: C07D 209/52, C07D 471/04

(54) **A process for preparing naphthyridones and intermediates**
Ein Verfahren zur Herstellung von Naphthyridonen und Zwischenprodukten
Un procédé de préparation de naphthyridones et intermédiaires

(30) Priority: 16.01.1998 US 71601 P
(43) Date of publication of application: 21.07.1999
(73) Proprietor: Pfizer Products Inc., Groton, CT 06340-5146 (US)
(72) Inventor: Chiu, Charles Kwok-fung, Attleboro, Massachusetts 02703 (US); Wint, Lewin Theophilus, Old Saybrook, Connecticut 06475 (US)
(74) Representative: Atkinson, Jonathan David Mark

(56) References cited:
- EP-A- 0 413 455
- WO-A-93/18001
- DE-A- 19 733 439
- US-A- 5 475 116
- T.F. BRAISH ET AL.: "Construction of the (1-alpha,5-alpha,6-alpha)-6-amino-3-azabic yclo(3.1.0.)hexane ring system" SYNLETT., no. 11, 1996, pages 1100-1102, XP002100147 STUTTGART DE

## Description

This invention relates to a process for preparing the naphthyridone carboxylic acid, trovafloxacin and derivatives thereof, and intermediates of use therein.

Trovafloxacin has the formula 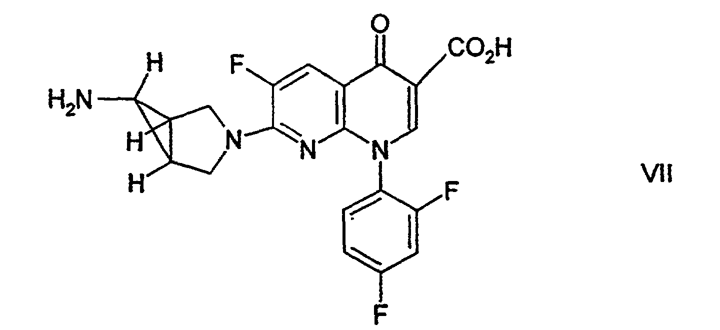 as disclosed in U.S. Patent No. 5,164,402. The patent also discloses processes for making the compound by using an intermediate of the formula 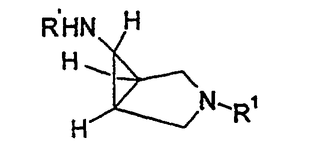 wherein R' is a nitrogen protecting group, such as tertiary butyloxycarbonyl.

U.S. Patent No. 5,475,116 discloses the preparation of other intermediates for use in preparing the naphthyridones of U.S. Patent No. 5,164,402.

WO93/18001 discloses the preparation of intermediates in the synthesis of quinoline antibiotics. More specifically, this citation relates to process for the preparation of intermediates in the synthesis of the quinoline antibiotic 7- (1α,5α,6α) - (6-amino-3-azabicyclo[3.1.0] hex-3-yl) -1(2, 4-difluorophenyl)-6-fluoro-1, 4-dihydro-4-oxo-1,8-naphthyridine -3-carboxylic acid and related antibiotic compounds.

Synlett number 11, 1996 pages 1100-1102 discloses the construction of the (1α,5α,6α) -6-amino-3-azabicyclo[3.1.0] hexane ring system using bromonitromethane. This citation discloses that the reaction of bromonitromethane with N-benzyl maleimide was attempted in the presence of a base but many bases failed to give any significant product. The authors of this citation did, however, find that DMTHP (dimethyl-1, 3, 4, 5-tetrahydropyrimidine) gave yields as high as 36% and thus used this base.

The present invention relates to a process for preparing a compound of the formula 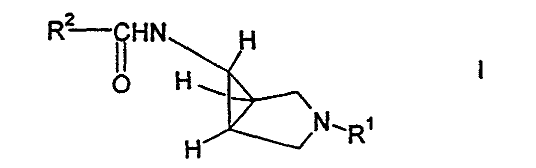 wherein R¹ is benzyl, wherein the phenyl of the benzyl may be substituted by one or more of C₁-C₆ alkyl, C₁-C₆ alkoxy, halo, nitro, amino or trifluoromethyl, and
R² is C₁-C₆ alkyl, trifluoromethyl, or phenyl which may be substituted by one or more of C₁-C₆ alkyl, C₁-C₆ alkoxyl halo, nitro, amino or trifluoromethyl, which comprises
(a) reducing a compound of the formula 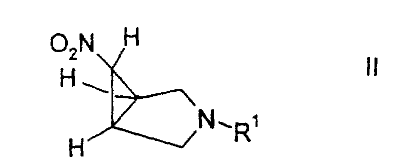 wherein R¹ is as defined above, in the presence of iron and a organic solvent under acidic conditions, and
(b) acylating the compound of formula III formed: 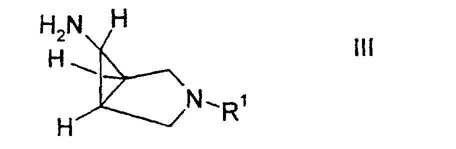 with an acylating agent of the formula R²C(O)X wherein R² is as defined above, and X is a leaving group.

In a prefered embodiment of the invention, the compound of formula III formed in step (a) is not isolated before acylation step (b).

The invention is further related to a process for preparing a compound of the formula 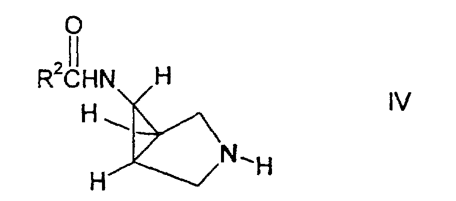 by debenzylating the compound of formula I wherein R¹ and R² are as defined above.

In a preferred embodiment, the debenzylation is carried out by reacting a compound of formula I with hydrogen and palladium catalyst in acetic acid and an organic solvent

The invention also relates to reacting a compound of the formula IV with a compound of the formula 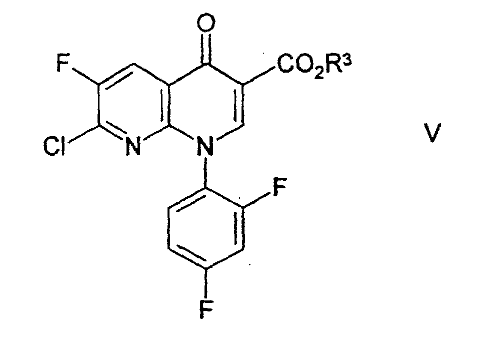 wherein R³ is C₁-C₆ alkyl, to form a compound of the formula 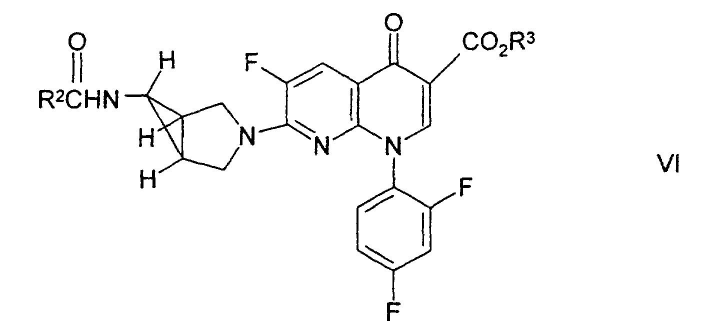 wherein R² is as defined above with reference to formula I.

The invention relates to hydrolyzing the compound of formula VI with methanesulfonic acid, water and an organic solvent to form the monomethanesulfonic acid salt of the compound of the formula VII, trovafloxacin.

The invention also relates to hydrolysis of the compound of formula VI with methanesulfonic acid and R³OH wherein R³ is as defined above to form the monomethanesulfonic acid salt of the compound of the formula

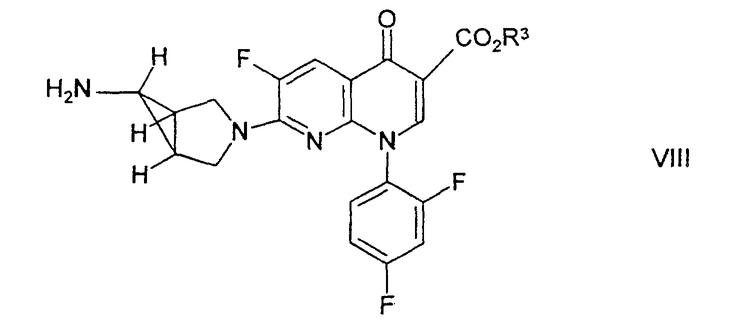

The invention further relates to the intermediates of the formulae 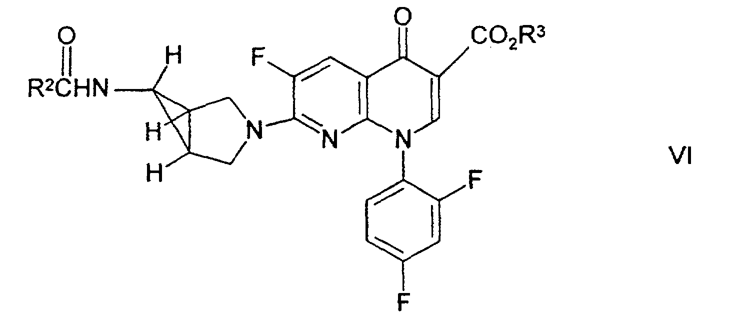 wherein R² is C₁-C₆ alkyl, trifluoromethyl, or phenyl which may be substituted by one or more of C₁-C₆ alkyl, C₁-C₆ alkoxy, halo, nitro, amino or trifluoromethyl,
R³ is C₁-C₆ alkyl

The term "alkyl", as used herein, includes saturated monovalent hydrocarbon radicals having straight or branched moieties, e.g. methyl, ethyl.

The term "alkoxy", as used herein, includes O-alkyl groups wherein "alkyl" is defined above.

The processes of the invention are depicted in the following reaction scheme. Unless indicated otherwise, R¹, R², R³ and X are as defined above.

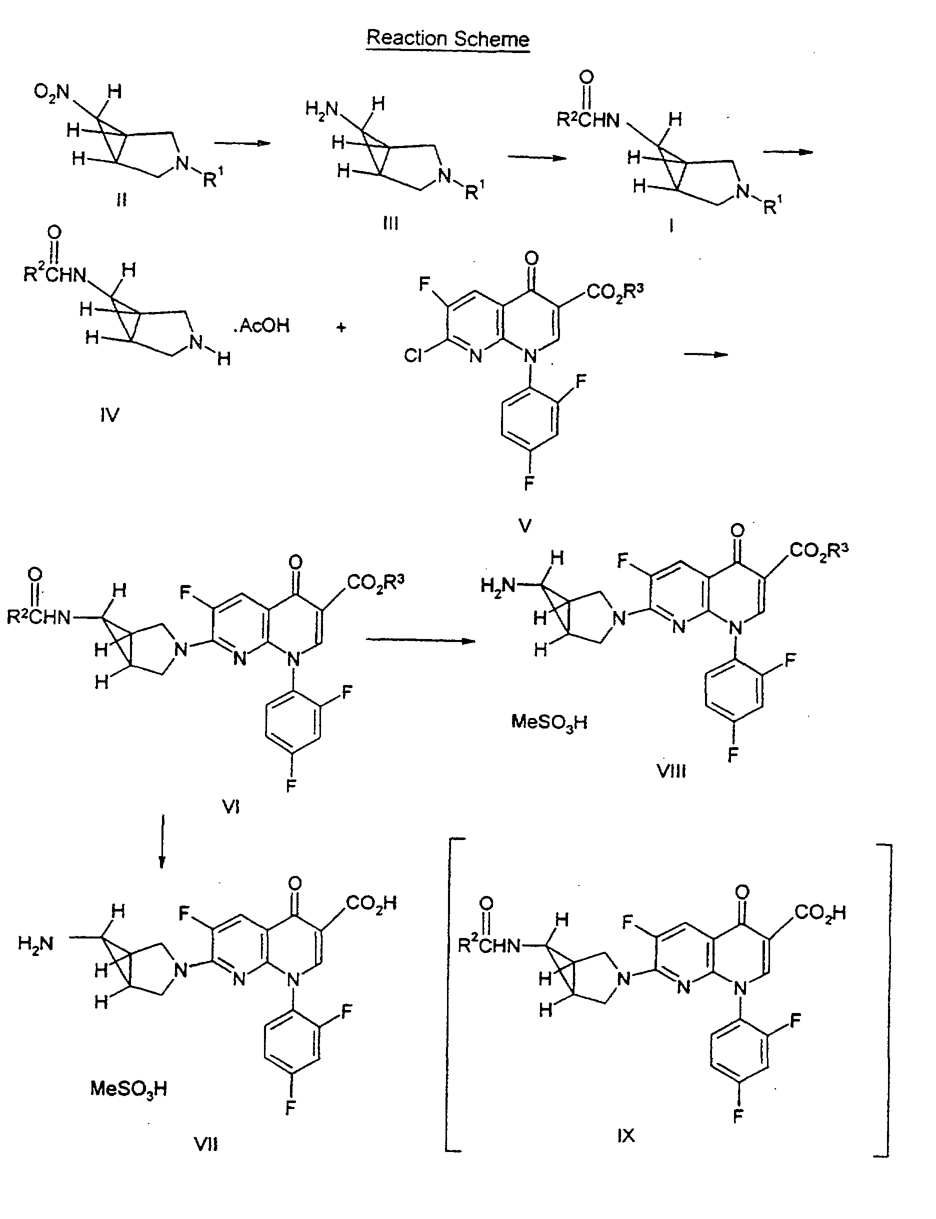

The compound of formula III is prepared from the corresponding compound of formula II by reduction in the presence of iron and an organic solvent under acidic conditions. The organic solvent is a C₁-C₆ alcohol, such as ethanol, or an ether such as tetrahydrofuran (THF), and preferably, an alcohol. The acidic conditions are obtained by use of a mineral acid, such as hydrochloric acid, or an organic acid, such as acetic acid (AcOH). Acetic acid is preferred since it generally results in increased yields.

The compound of formula III may then be isolated from the reaction mixture or may be reacted further in situ, without isolation from the reaction mixture. In either case, the further processing is by acylation with an acylating agent of the formula R²C(O)X to form the compound of formula I. The leaving group X is conveniently a halogen, such as chloro, or the acetoxy group. If the compound of formula III is first isolated, then the acylation may be conducted under conventional acylating conditions, for instance, in the presence of an organic solvent of the type discussed above.

The compound of formula I is subjected to debenzylation to form the compound of formula IV. It is understood that in the context of the invention, debenzylation includes removal of R¹ wherein R¹ is benzyl or substituted benzyl. The reaction proceeds in accordance with conventional debenzylation of tertiary nitrogen, conveniently by use of hydrogen and palladium catalyst in acetic acid, and in an organic solvent. The organic solvent may be a C₁-C₆ alcoholic solvent, such as ethanol, ethyl acetate, THF or water, or a mixture thereof, such as ethanol and water.

The compound of formula VI is obtained by coupling the corresponding compound of formula IV with the bicyclic intermediate ester of formula V. This coupling reaction may be conducted with or without a solvent. The solvent, when used, must be inert under the reaction conditions. Suitable solvents are ethyl acetate, acetonitrile, tetrahydrofuran, ethanol, chloroform, dimethylsulfoxide, pyridine, and water, and mixtures thereof.

The reaction temperature usually ranges from about 20°C. to about 150°C.

The reaction may advantageously be carried out in the presence of an acid acceptor such as an inorganic or organic base, e.g. an alkali metal or alkaline earth metal carbonate or bicarbonate, or a tertiary amine, e.g. triethylamine, pyridine or picoline.

The mesylate salt of the compound of formula VII, trovafloxacin, is formed by hydrolysis of the compound of formula VI with methanesulfonic acid, water and an organic solvent Examples of suitable organic solvents include a C₁-C₆ alcohol, acetone, dimethoxy ethane, glyme, THF, N-methyl-pyrrolidinone, and water, and mixtures thereof.

The mesylate salt of the compound of formula VIII is obtained by hydrolysis of the compound of formula VI with methanesulfonic acid and a C₁-C₆ alcohol of the formula R³OH, for example ethanol. The compound of formula VIII is an intermediate in the preparation of the mesylate salt of a prodrug of trovafloxacin wherein the amino group is substituted by an amino acid or a polypeptide, e.g. dipeptide, as disclosed in U.S. Patent No. 5,164,402.

The compound of formula IX in the Reaction Scheme is the intermediate formed in the reaction from compound VI to VII.

The compound of formula VII and the mesylate salt thereof (the active compounds) are useful in the treatment of bacterial infections of broad spectrum, particularly the treatment of gram-positive bacterial strains.

The active compounds may be administered alone, but will generally be administered in admixture with a pharmaceutical carrier selected with regard to the intended route of administration and standard pharmaceutical practice. For example, they can be administered orally or in the form of tablets containing such excipients as starch or lactose, or in capsules either alone or in admixture with excipients, or in the form of elixirs or suspensions containing flavoring or coloring agents. In the case of animals, they are advantageously contained in an animal feed or drinking water in a concentration of 5-5000 ppm, preferably 25-500 ppm. They can be injected parenterally, for example, intramuscularly, intravenously or subcutaneously. For parenteral administration, they are best used in the form of a sterile aqueous solution which can contain other solutes, for example, enough salt or flucose to make the solution isotonic. In the case of animals, compounds can be administered intramuscularly or subcutaneously at dosage levels of about 0.1-50 mg/kg/day, advantageously 0.2-10 mg/kg/day given in a single daily dose or up to 3 divided doses.

The invention also provides pharmaceutical compositions comprising an antibacterially effective amount of a compound of the formula (I) together with a pharmaceutically acceptable diluent or carrier.

The compounds of the invention can be administered to humans for the treatment of bacterial diseases by either the oral or parenteral routes, and may be administered orally at dosage levels of about 0.1 to 500 mg/kg/day, advantageously 0.5-50 mg/kg/day given in a single dose or up to 3 divided doses. For intramuscular or intravenous administration, dosage levels are about 0.1-200 mg/kg/day, advantageously 0.5-50 mg/kg/day. While intramuscular administration may be a single dose or up to 3 divided doses, intravenous administration can include a continuous drip. Variations will necessarily occur depending on the weight and condition of the subject being treated and the particular route of administration chosen as will be known to those skilled in the art.

The following Examples illustrate the invention. The abbreviations used mean the following: GC=gas chromatography; MS=mass spectometry; TLC=thin layer chromatography, HPLC = high performance liquid chromatography; LCMS = liquid chromatography mass spectometry; and NMR = nuclear magnetic resonance.

### Example 1

### (1α, 5α, 6α)-6-Acetamido-3 -benzyl-3-azabicyclo[3.1.0 ]hexane

A 3-necked round bottom flask, equiped with a thermometer, a overhead stirrer and a condenser with nitrogen purge, was charged with 768 g of nitrocyclopropane, 5.75 L of isopropanol (7.5 volumes), 1.79 L of acetic acid (9.1 equivalents) and 1153 g of iron powder (6 equivalents). The reaction mixture was heated at 50°C until the reaction was completed by GC/MS analysis (about 6 hours). 448 mL of acetic anhydride (1.4 equivalents) was added and stirred at 50°C for 15 minutes before cooling. The reaction mixture was diluted with 8 L isopropanol (10.5 volumes) and stirred for 30 minutes. The residual iron was filtered off and the cake washed with 11.25 L of isopropanol (15 volumes). The isopropanol solution was concentrated in vacuo to an oil, 18 L of dichloroethane (24 volumes) was added before bringing the pH to 12 with 8.8 L of 5% sodium hydroxide solution (about 12 volumes). The layers were separated and the separated organic layer was dried by magnesium sulfate. The resulting dark amber oil was treated with 7.5 L of hexanes (10 volumes) and granulated at 25°C before collecting the product as a white solid. Drying at 50°C under vacuum gave 610 g of the title compound (77 % yield). Analysis was done by GC/MS, NMR and TLC.

### Example 2

### (1α, 5α, 6α)-6-Acetamido-3-azabicyclo[3.1.0]hexane

A Parr Bottle was charged with 150 g of the compound of Example 1, 112 mL of acetic acid (3 equivalents), 1.5 L of methanol (10 volumes) and 15 g of (10% by wt. 50% wet) Pd/C catalyst (0.1 equivalent). The bottle was purged with nitrogen and then brought to 50 psi pressure with hydrogen. The mixture was shaken for 48 hours and recharged with catalyst as necessary during the debenzylation reaction. After TLC indicated that the reaction was complete, the catalyst was filtered off, and the filtrate was concentrated in vacuo to an oil. 3 L of ethyl acetate (20 volumes) was added to the oil, and granulated for an hour. The solid was collected by filtration and dried under vacuum at 50°C to provide 107 g of the title compound (82 % yield) as the acetic acid salt.

### Example 3

### (1α, 5α, 6α)-7-(6-acetamido-3-azabicyclo[3.1.0]hex-3-yl)-1-(2,4-difluorophenyl)-6-fluoro-1,4-dihydro-4-oxo-1,8-naphthyridine-3-carboxylic acid, ethyl ester

A reaction flask was charged with 241.9 g of 7-chloro-6-fluoro-1,4-dihydro-4-oxo-1,8-naphthyridine-3-carboxylic acid ethyl ester, 151.6 g of the acetic acid salt of the compound of Example 2 (1.2 equivalents), 2661mL of ethyl acetate (11 volumes) and 220 mL of triethylamine (2.5 equivalents). The mixture was heated at refluxing temperature under nitrogen for 6 hours monitored by HPLC or LCMS. After the reaction was completed, the reaction mixture was cooled to ambient temperature. Water (11 volumes) was added and the biphasic mixture was stirred for 17 hours. The white solid was collected by filtration, washed with 2661 mL of water (12 volumes) and oven dried at 50°C to provide 292 g of the title compound (95 % yield).

### Example 4

In a reaction flask, 220 g of the compound of Example 3, 1.76 L of n-butanol (8 volumes), 1.54 L of water (7 volumes) and 141 mL of 70% methanesulfonic acid (3.0 equivalents) were mixed. The mixture was heated at reflux for 21 hours, and the reaction was monitored by HPLC or LCMS. After complete reaction, the mixture was cooled to 50°C and filtered to make it speck-free. The filtrated was cooled to 0-5°C and granulated for 2 hours. The solid was collected by filtration, washed with 220 mL of water (1 volume) and 660 mL n-butanol (3 volumes). The wet cake was mixed with 660 mL of n-butanol (3 volumes), seeded with 0.1 gm of the desired polymorph and heated to 95-100°C. After complete polymorph conversion, in approximately 2 hours, the mixture was cooled to ambient temperature. The solid was filtered, washed with 100 mL of n-butanol (0.5 volumes) and dried in a nitrogen atmosphere to provide 200 g of (1α, 5α, 6α)-7-(6-amino-3-azabicyclo[3.1.0]hex-3-yl)-1-(2,4-difluorophenyl)-6-fluoro-1,4-dihydro-4-oxo-1,8-naphthyridine-3-carboxylic acid, monomethanesulfonate (87% yield).

### Example 5

0.8 mL of methanesulfonic acid (2.7 equivalents) was added dropwise to a solution of 2.2 g of the compound of Example 3 in 10 mL of ethanol (4.5 volumes). The resulting reaction mixture was heated at refluxing temperature for 40 hours, monitored by GCMS. After the reaction was completed, it was diluted with ethyl acetate (20 mL) and washed with (3x 10ml) 1M sodium hydroxide solution. The organic layer was separated, dried over anhydrous magnesium sulphate and filtered. The filtrate was concentrated in vacuo to provide 1.37 g of (1α, 5α, 6α)-7-(6-amino-3-azabicyclo[3.1.0]hex-3-yl)-1-(2,4-difluorophenyl)-6-fluoro-1,4-dihydro-4-oxo-1,8-naphthyridine-3-carboxylic acid ethyl ester, monomethanesulfonate (96% yield).

## Claims

1. A process for preparing a compound of the formula 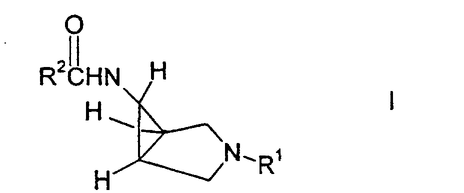 wherein
R¹ is benzyl, wherein the phenyl of the benzyl may be substituted by one or more of C₁-C₆ alkyl, C₁-C₆ alkoxy, halo, nitro, amino or trifluoromethyl, and
R² is C₁-C₆ alkyl, trifluoromethyl, or phenyl which may be substituted by one or more of C₁-C₆ alkyl, C₁-C₆ alkoxy, halo, nitro, amino or trifluoromethyl, which comprises
(a) reducing a compound of the formula 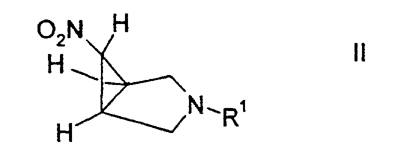 wherein R¹ is as defined above, in the presence of iron and a organic solvent under acidic conditions, and
(b) acylating the compound of formula III formed: 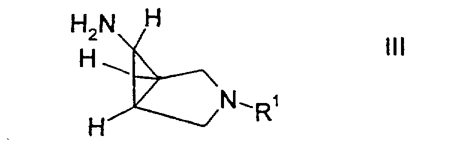 with an acylating agent of the formula R²C(O)X wherein R² is as defined above, and X is a leaving group.

2. A process according to claim 1 wherein the compound of the formula III formed in step (a) is not isolated before acylation step (b).

3. A process according to claim 1 or 2 wherein the compound of formula I wherein R¹ is as defined in claim 1, is subjected to debenzylation to form the compound of the formula

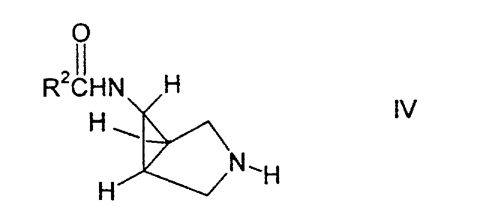

4. A process according to claim 3 wherein the debenzylation is by reaction with hydrogen and palladium catalyst in acetic acid and an organic solvent

5. A process according to claim 3 or 4 further comprising reacting the compound of formula IV with a compound of the formula 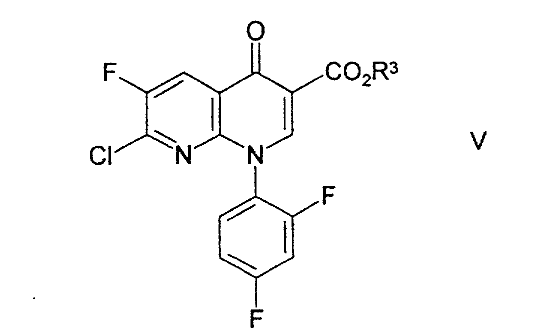 wherein R³ is C₁-C₆ alkyl, to form a compound of the formula 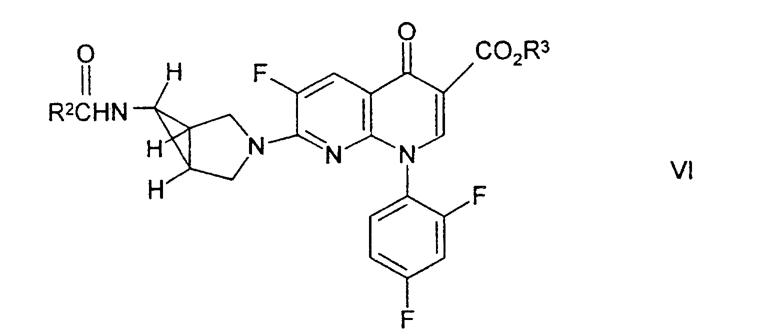 wherein R² is as defined in claim 1

6. A process according to claim 5 further comprising hydrolysis of the compound of formula VI with methanesulfonic acid, water and an organic solvent to form the monomethanesulfonic acid salt of the compound of the formula

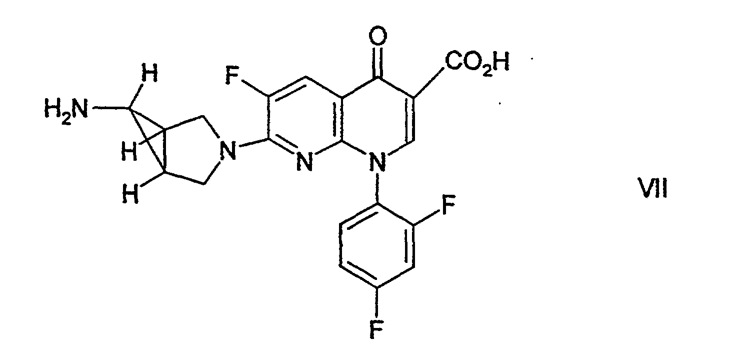

7. A process according to claim 5 or 6 further comprising hydrolysis of the compound of formula VI with methanesulfonic acid and R³OH wherein R³ is as defined in claim 5 to form the monomethanesulfonic acid salt of the compound of the formula

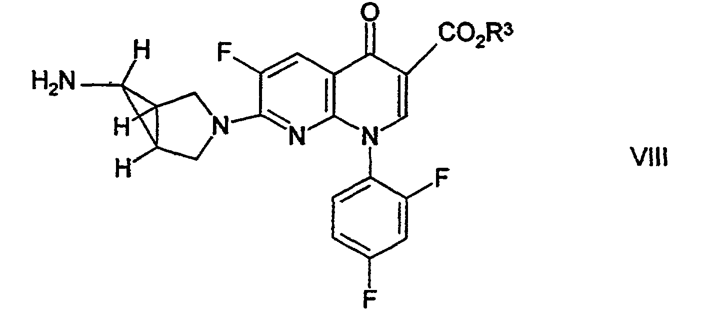

8. A process according to claims 1-4 for the preparation of a compound of the formula 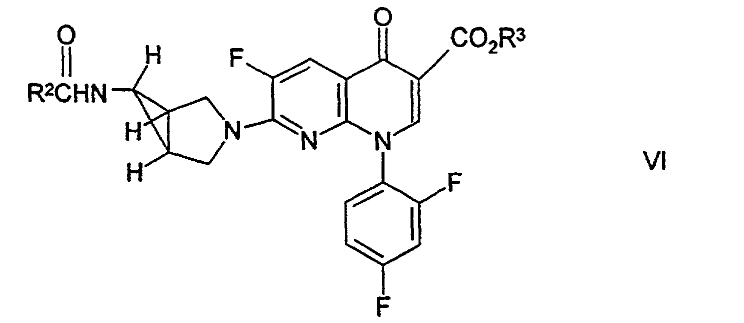 wherein R² is C₁-C₆ alkyl, trifluoromethyl, or phenyl which may be substituted by one or more of C₁-C₆ alkyl, C₁-C₆ alkoxy, halo, nitro, amino or trifluoromethyl, and
R³ is C₁-C₆ alkyl, which comprises reacting a compound of the formula 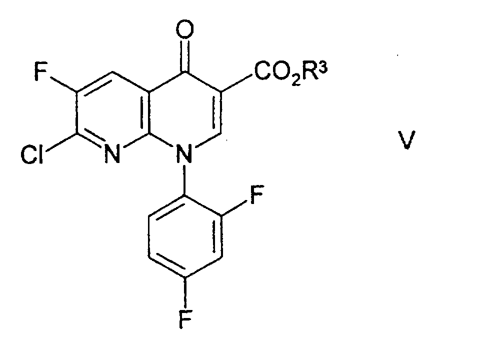 with a compound of the formula

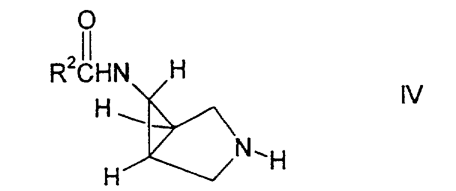

9. A process according to claim 8, further comprising hydrolysis of the compound of formula VI with methanesulfonic acid, water and an organic solvent to form the monomethanesulfonic acid salt of the compound of the formula

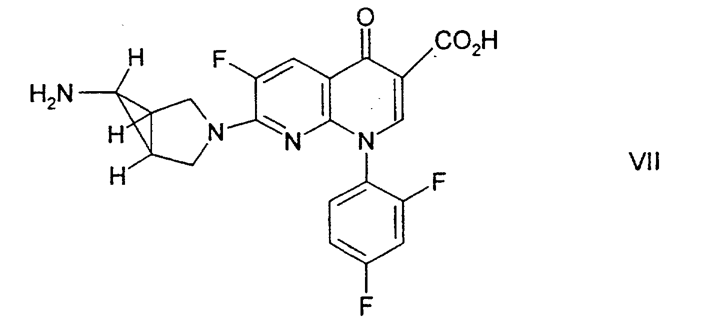

10. A process according to claim 8, further comprising hydrolysis of the compound of formula VI with methanesulfonic acid and R³OH wherein R³ is as defined in claim 5 to form the monomethanesulfonic acid salt of the compound of the formula

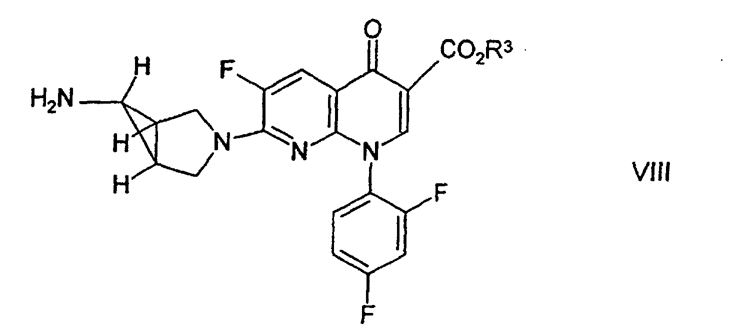

11. A compound of the formula 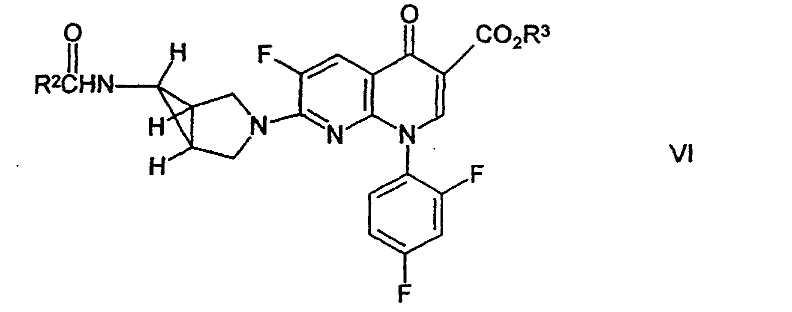 wherein
R² is C₁-C₆ alkyl, trifluoromethyl, or phenyl which may be substituted by one or more of C₁-C₆ alkyl, C₁-C₆ alkoxy, halo, nitro, amino or trifluoromethyl, and
R³ is C₁-C₆ alkyl.

## Patentansprüche

1. Verfahren zur Herstellung einer Verbindung der Formel 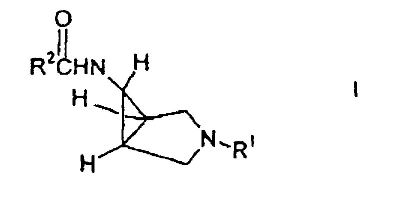 worin
R¹ ein Benzylrest ist, wobei der Phenylanteil des Benzylrestes mit einer oder mehreren C₁-C₆-Alkyl-, C₁-C₆-Alkoxy-, Halogen-, Nitro-, Amino- oder Trifluormethylgruppen substituiert sein kann und
R² ein C₁-C₆-Alkyl-, Trifluormethyl- oder Phenylrest ist, der mit einer oder mehreren C₁-C₆-Alkyl-, C₁-C₆-Alkoxy-, Halogen-, Nitro-, Amino- oder Trifluormethylgruppen substituiert sein kann, das umfasst, dass
(a) eine Verbindung der Formel 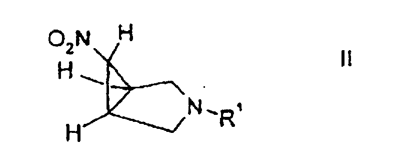 worin R¹ wie oben definiert ist, in Gegenwart von Eisen und einem organischen Lösungsmittel unter sauren Bedingungen reduziert wird und
(b) die gebildete Verbindung der Formel III 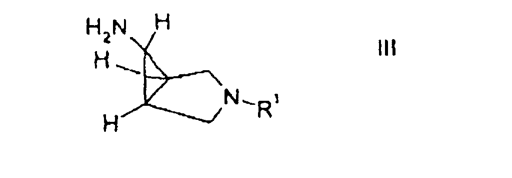 mit einem Acylierungsmittel der Formel R²C(O)X, worin R² wie oben definiert ist und X eine Abgangsgruppe ist, acyliert wird.

2. Verfahren nach Anspruch 1, wobei die Verbindung der Formel III, die in Stufe (a) gebildet wird, nicht vor der Acylierungsstufe (b) isoliert wird.

3. Verfahren nach Anspruch 1 oder Anspruch 2, wobei die Verbindung der Formel I, worin R¹ wie in Anspruch 1 definiert ist, einer Debenzylierung unterworfen wird, um eine Verbindung der Formel 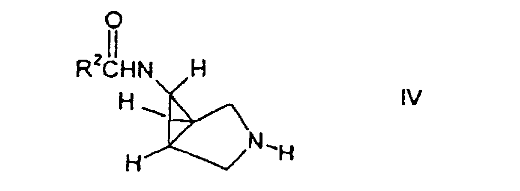 zu bilden.

4. Verfahren nach Anspruch 3, wobei die Debenzylierung durch Reaktion mit Wasserstoff und Palladium als Katalysator in Essigsäure und einem organischen Lösungsmittel erfolgt.

5. Verfahren nach Anspruch 3 oder Anspruch 4, das weiter umfasst, dass die Verbindung der Formel IV mit einer Verbindung der Formel 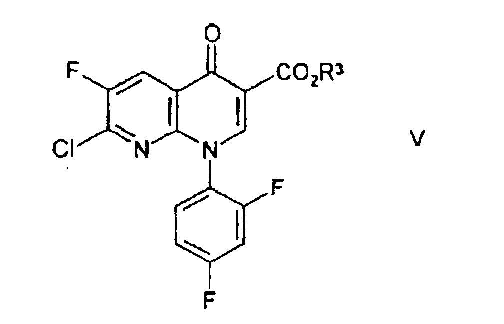 umgesetzt wird, worin R³ ein C₁-C₆-Alkylrest ist, um eine Verbindung der Formel 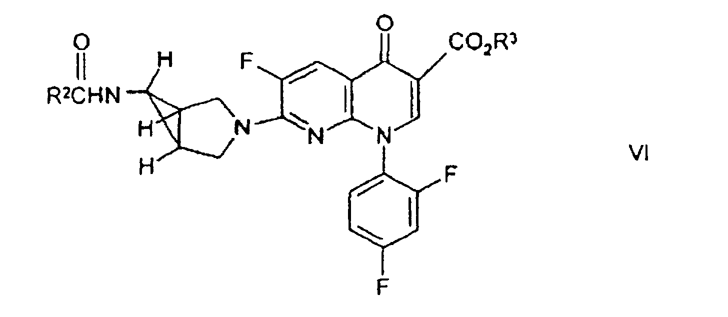 zu bilden, worin R² wie in Anspruch 1 definiert ist.

6. Verfahren nach Anspruch 5, das weiter die Hydrolyse der Verbindung der Formel VI mit Methansulfonsäure, Wasser und einem organischen Lösungsmittel umfasst, um das Monomethansulfonsäuresalz der Verbindung der Formel 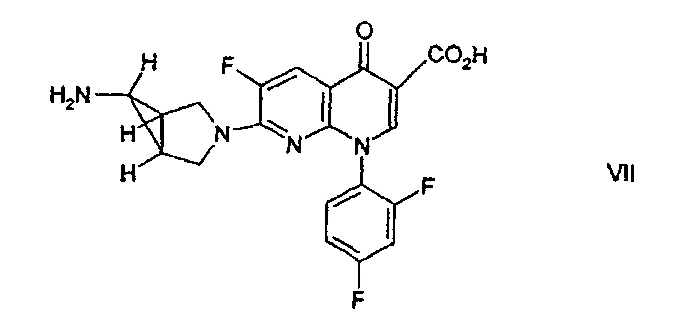 zu bilden.

7. Verfahren nach Anspruch 5 oder Anspruch 6, das weiter die Hydrolyse der Verbindung der Formel VI mit Methansulfonsäure und R³OH umfasst, wobei R³ wie in Anspruch 5 definiert ist, um das Monomethansulfonsäuresalz der Verbindung der Formel 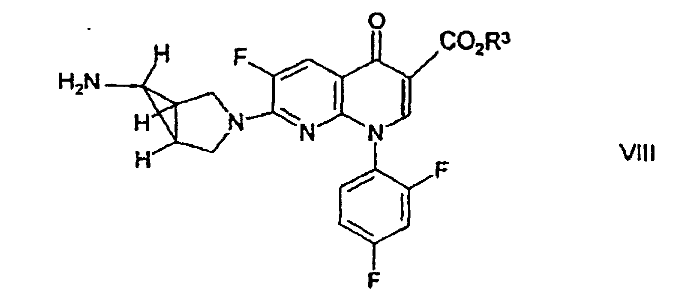 zu bilden.

8. Verfahren nach einem der Ansprüche 1 bis 4 zur Herstellung einer Verbindung der Formel 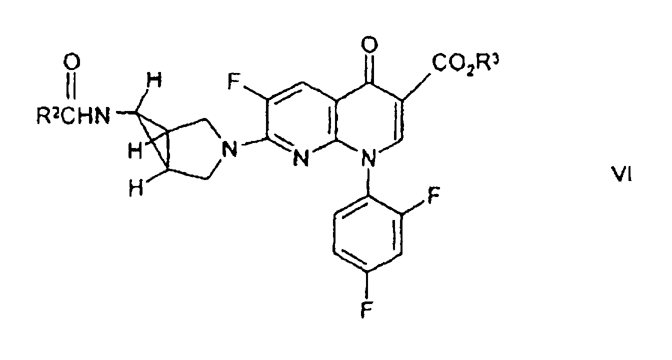 worin
R² ein C₁-C₆-Alkyl-, Trifluormethyl- oder Phenylrest ist, der mit einer oder mehreren C₁-C₆-Alkyl-, C₁-C₆-Alkoxy-, Halogen-, Nitro-, Amino- oder Trifluormethylgruppen substituiert sein kann und
R³ ein C₁-C₆-Alkylrest ist, das umfasst, dass eine Verbindung der Formel 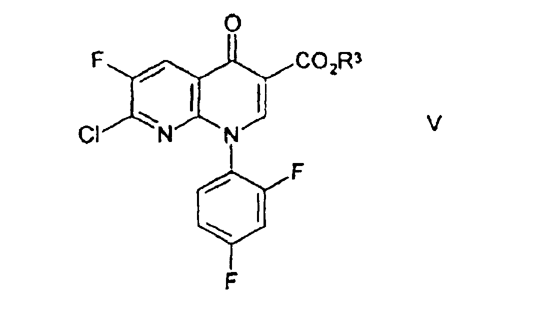 mit einer Verbindung der Formel 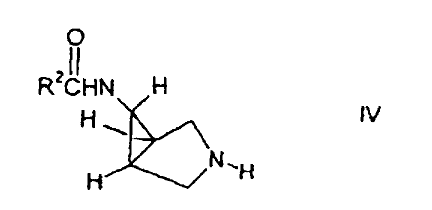 umgesetzt wird.

9. Verfahren nach Anspruch 8, das weiter die Hydrolyse der Verbindung der Formel VI mit Methansulfonsäure, Wasser und einem organischen Lösungsmittel umfasst, um das Monomethansulfonsäuresalz der Verbindung der Formel 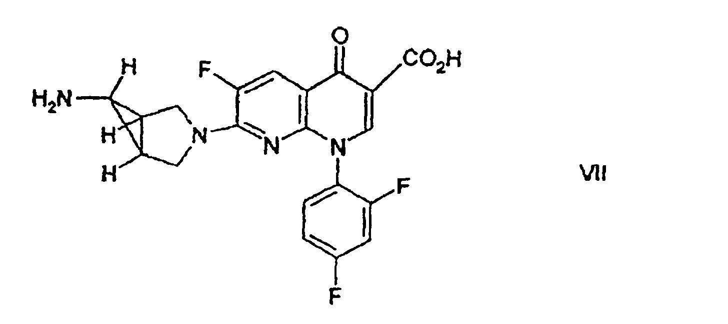 zu bilden.

10. Verfahren nach Anspruch 8, das weiter die Hydrolyse der Verbindung der Formel VI mit Methansulfonsäure und R³OH umfasst, wobei R³ wie in Anspruch 5 definiert ist, um das Monomethansulfonsäuresalz der Verbindung der Formel 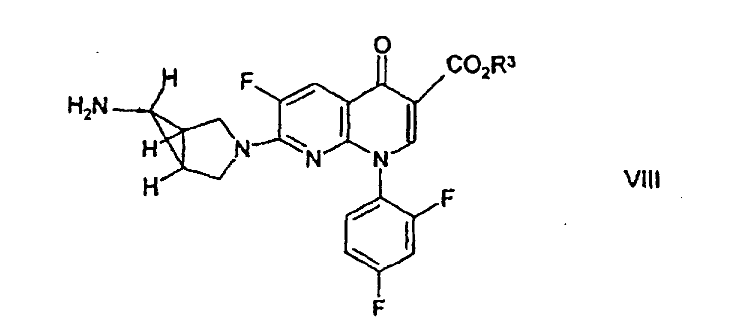 zu bilden.

11. Verbindung der Formel 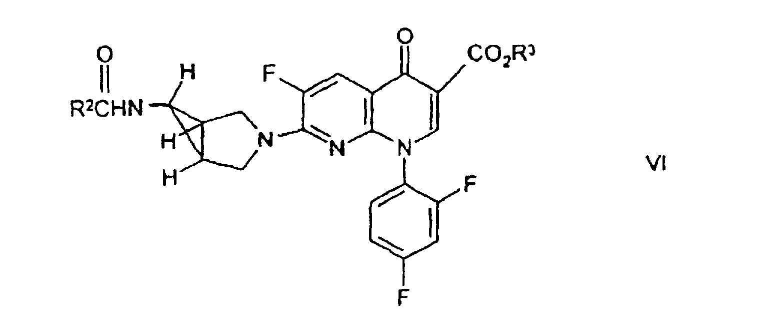 worin
R² ein C₁-C₆-Alkyl-, Trifluormethyl- oder Phenylrest ist, der mit einer oder mehreren C₁-C₆-Alkyl-, C₁-C₆-Alkoxy-, Halogen-, Nitro-, Amino- oder Trifluormethylgruppen substituiert sein kann und
R³ ein C₁-C₆-Alkylrest ist.

## Revendications

1. Procédé pour la préparation d'un composé de formule 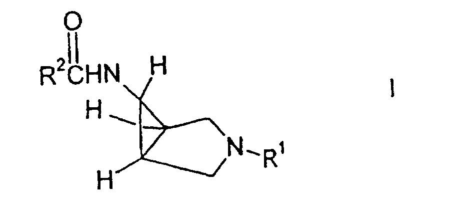 dans laquelle
R¹ représente un groupe benzyle, dans lequel le groupe phényle du groupe benzyle peut être substitué avec un ou plusieurs substituants alkyle en C₁ à C₆, alkoxy en C₁ à C₆, halogéno, nitro, amino ou trifluorométhyle, et
R² représente un groupe alkyle en C₁ à C₆, un groupe trifluorométhyle, ou un groupe phényle qui peut être substitué avec un ou plusieurs substituants alkyle en C₁ à C₆, alkoxy en C₁ à C₆, halogéno, nitro, amino ou trifluorométhyle, qui comprend
(a) la réduction d'un composé de formule 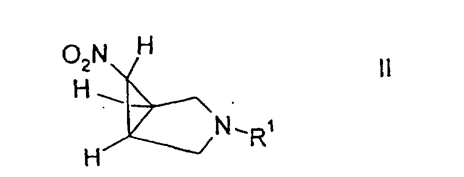 dans laquelle R¹ répond à la définition précitée, en présence de fer et d'un solvant organique dans des conditions acides, et
(b) l'acylation du composé de formule III formé : 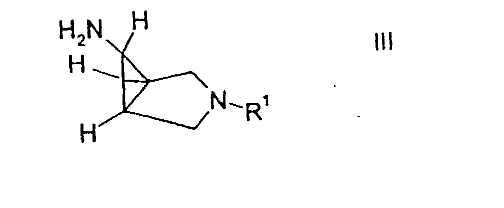 avec un agent d'acylation de formule R²C(O)X dans laquelle R² répond à la définition précitée et X représente un groupe partant.

2. Procédé suivant la revendication 1, dans lequel le composé de formule III formé dans l'étape (a) n'est pas isolé avant l'étape d'acylation (b).

3. Procédé suivant la revendication 1 ou 2, dans lequel le composé de formule I dans laquelle R¹ répond à la définition suivant la revendication 1 est soumis à une débenzylation pour former le composé de formule

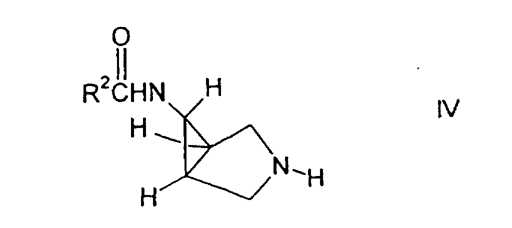

4. Procédé suivant la revendication 3, dans lequel la débenzylation est effectuée par réaction avec de l'hydrogène et un catalyseur au palladium dans l'acide acétique et un solvant organique.

5. Procédé suivant la revendication 3 ou 4, comprenant en outre la réaction du composé de formule IV avec un composé de formule 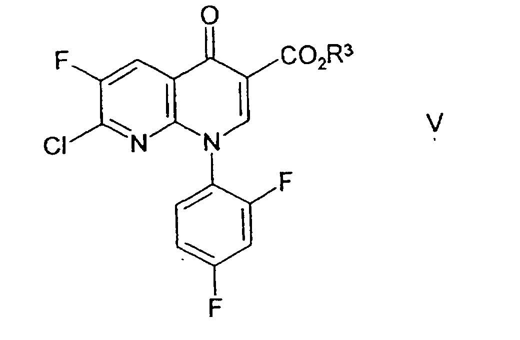 dans laquelle R³ représente un groupe alkyle en C₁ à C₆, pour former un composé de formule 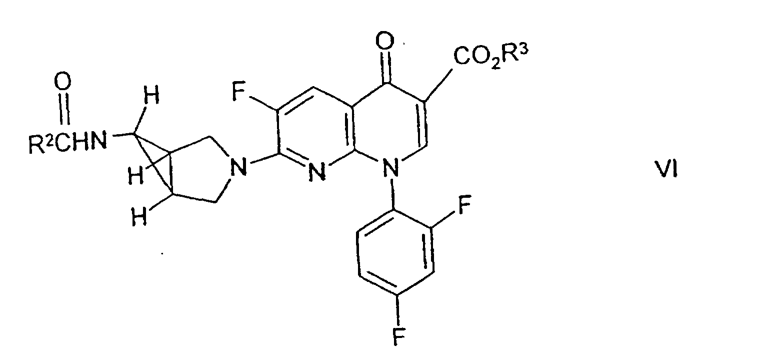 dans laquelle R² répond à la définition suivant la revendication 1.

6. Procédé suivant la revendication 5, comprenant en outre l'hydrolyse du composé de formule VI avec de l'acide méthanesulfonique, de l'eau et un solvant organique pour former le sel d'acide monométhanesulfonique du composé de formule

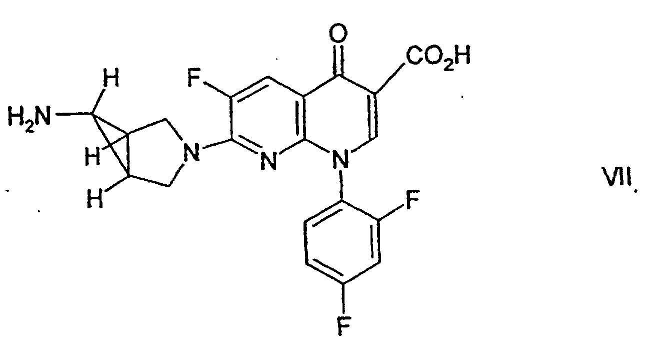

7. Procédé suivant la revendication 5 ou 6, comprenant en outre l'hydrolyse du composé de formule VI avec de l'acide méthanesulfonique et un composé de formule R³OH dans laquelle R³ répond à la définition suivant la revendication 5 pour former le sel d'acide monométhanesulfonique du composé de formule

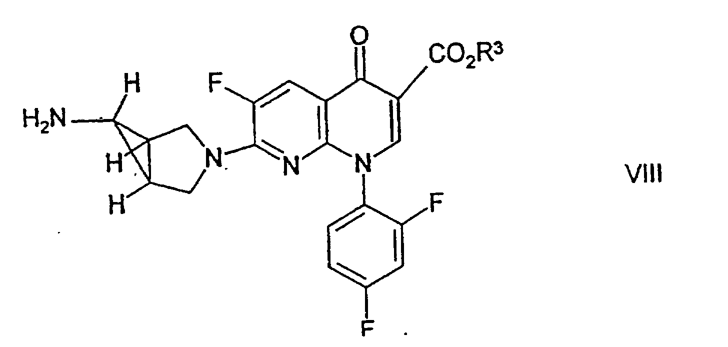

8. Procédé suivant les revendications 1 à 4 pour la préparation d'un composé de formule 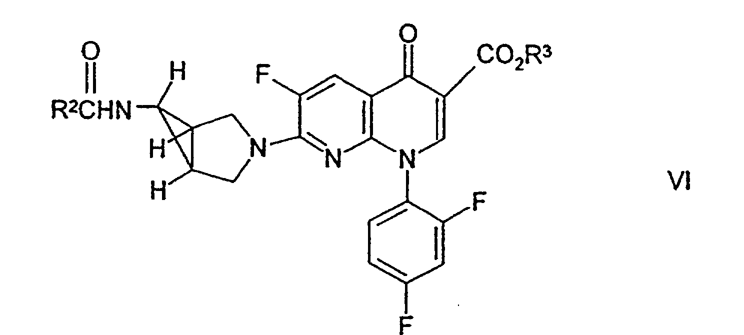 dans laquelle R² représente un groupe alkyle en C₁ à C₆, un groupe trifluorométhyle, un groupe phényle qui peut être substitué avec un ou plusieurs substituants alkyle en C₁ à C₆, alkoxy en C₁ à C₆, halogéno, nitro, amino ou trifluorométhyle, et
R³ représente un groupe alkyle en C₁ à C₆, qui comprend la réaction d'un composé de formule 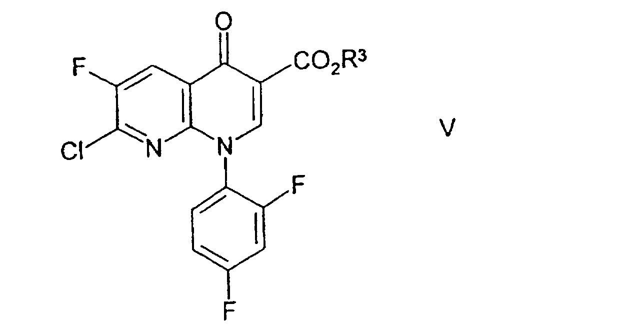 avec un composé de formule

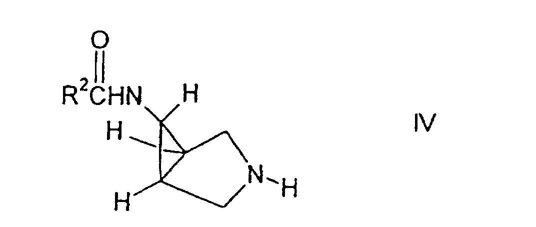

9. Procédé suivant la revendication 8, comprenant en outre l'hydrolyse du composé de formule VI avec de l'acide méthanesulfonique, de l'eau et un solvant organique pour former un sel d'acide monométhanesulfonique du composé de formule

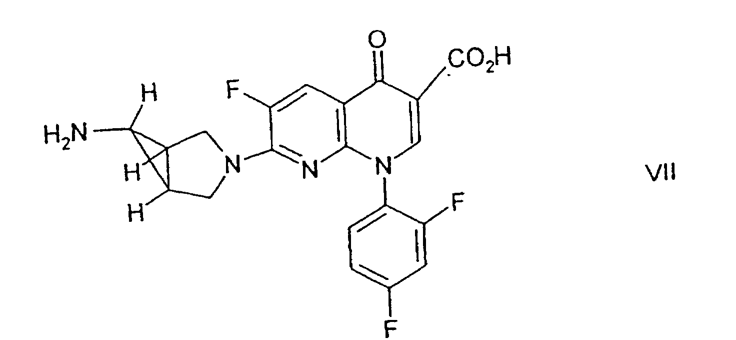

10. Procédé suivant la revendication 8, comprenant en outre l'hydrolyse du composé de formule VI avec de l'acide méthanesulfonique et un composé de formule R³OH dans laquelle R³ répond à la définition suivant la revendication 5 pour former le sel d'acide monométhanesulfonique du composé de formule

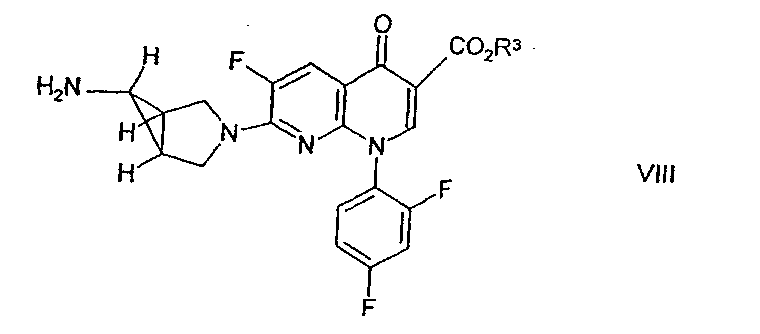

11. Composé de formule 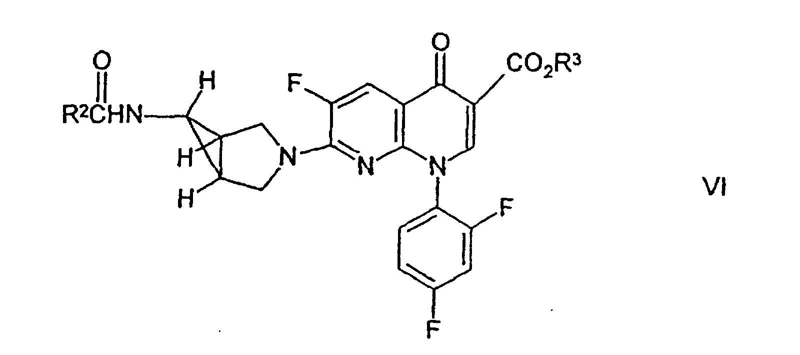 dans laquelle
R² représente un groupe alkyle en C₁ à C₆, un groupe trifluorométhyle, ou un groupe phényle qui peut être substitué avec un ou plusieurs substituants alkyle en C₁ à C₆, alkoxy en C₁ à C₆, halogéno, nitro, amino ou trifluorométhyle, et
R³ représente un groupe alkyle en C₁ à C₆.
